# EUROPEAN PATENT SPECIFICATION

(11) **EP 2 854 619 B1**
(45) Date of publication and mention of the grant of the patent: **23.08.2017**
(21) Application number: 13737396.5
(22) Date of filing: 27.05.2013
(51) Int. Cl.: A61B 1/05, A61B 1/00, A61B 1/06

(54) **ENDOSCOPE**
ENDOSKOP
ENDOSCOPE

(30) Priority: 31.05.2012 IT MI20120948
(43) Date of publication of application: 08.04.2015
(73) Proprietor: Politecnico di Milano, 20133 Milano (IT)
(72) Inventor: CERVERI, Pietro, I-20133 Milan (IT); ZAZZARINI, Cynthia, I-20133 Milan (IT)
(74) Representative: Perani, Aurelio
(86) International application number: PCT/IB2013/054370
(87) International publication number: WO 2013/179207

(56) References cited:
- EP-A1- 1 836 946
- WO-A2-2010/014792
- US-A1- 2007 149 855
- US-A1- 2010 063 361

## Description

### TECHNICAL FIELD

The present invention relates to a miniaturized apparatus for endoscopic vision, particularly an endoscopic camera providing an interior view of a body and adapted for medical use, for instance in the fields of diagnostics and mini-invasive surgery. As used in this disclosure and the accompanying claims, the term "endoscope" will be used to designate a miniaturized apparatus for endoscopic vision.

### BACKGROUND OF THE INVENTION

Endoscopes are typically introduced in natural orifices or specially formed apertures, for providing an interior view of a body.

In medical use, endoscopes are used to provide an interior view of cavities, e.g. abdominal and thoracic cavities, either for diagnostics or for displaying the surgical field. In 1806 Philip Bozzini built the prototype of an instrument that had the purpose of providing a view of the internal organs of a human body and, after further developments, such instrument was first introduced into a patient by a French urologist in 1853. From that time on, endoscopes have been continuously developed, from the most simple and invasive gastroscopes that were built at about the middle of the last century, to the later introduction of optical fibers as visual components. Recent progresses in the electronic field have further improved endoscopes and endoscopic cameras are now available, which have a small size and can magnify and adequately illuminate the cavity to be explored.

The most recent endoscopes of this type have a substantially cylindrical body, which is designed to be introduced in the body to be explored and are equipped with optics having variable focus and magnification. The lenses of the optical system have such lengths as to allow the image to be focused on a sensor external to the cylindrical body (and external to the patient's body). The external sensor is focused by connecting the optics and the sensor by optical fibers. Optical fibers further allow the cylindrical body to be adequately illuminated, to illuminate the cavity to be explored. These optical fibers are introduced into a special flexible channel, that protects them during use. Actuators, usually electromechanical actuators are provided to move the lenses of the optical system, and are powered by appropriate power sources located outside the cylindrical body.

While prior art endoscopes, namely prior art endoscopic cameras are highly reliable and efficient, they still suffer from certain drawbacks.

For example, the optical fibers that connect the optical system with the external sensor do not allow introduction of the endoscope along particularly tortuous paths, i.e. having very narrow bending radiuses, due to the poor flexibility of optical fibers.

Also, particularly great lengths are required for the lenses to focus the image on the remote sensor (for the relevant applications), which leads to endoscopes with large longitudinal dimensions.

Furthermore, in certain cases the use of electromechanical actuators to move the lenses of the optical system might turn out to be dangerous for the patient, because certain actuators that are used in prior art endoscopes have supply voltages of about 40 V (which is a hazardous voltage if it is transmitted to the internal organs of the patient).

In this context, the technical purpose of the present invention is to provide an endoscope that differs from those of the prior art.

Particularly, one object of the present invention is to provide an endoscope that has a small size, in terms of both transverse and longitudinal dimensions.

A further object of the present invention is to provide an endoscope that can be introduced into its intended site through particularly tortuous paths having large bending radiuses.

Yet another object of the present invention is to provide an endoscope that is easy to use.

A further object of the present invention is to provide an endoscope that is safe for the patient.

EP 1 836 946 A1 discloses a device according to the preamble of claim 1.

### SUMMARY OF THE INVENTION

According to the present invention, the technical purpose and the intended objects are fulfilled by an endoscope as claimed in one or more of the annexed claims.

In particular, it is disclosed an endoscope comprising:
- a substantially cylindrical housing, which is designed to be introduced into a body to be examined;
- an optical focusing and zooming device contained in said housing;
- motion imparting members contained in said housing, operating on said optical device and designed to move part of said optical device for its focusing and zooming operation;
- transducer members located in said housing downstream from said optical device and designed to convert an optical flow focused by said optical device into an electric signal;
- lighting members comprising at least one light source located in the housing;
- a control and monitoring unit, which is designed to receive signals representative of optical zoom degrees and is operably connected to said motion imparting members to actuate said motion imparting members according to the received optical zoom-representative signals;
- said optical device comprising a first set of lenses designed to cause an input optical flow to converge toward a second set of lenses, said second set of lenses being located downstream from said first set of lenses and being adapted to be moved by said motion imparting means toward and away from said first optical set and to change the overall optical zoom; a third set of lenses located downstream from said second set of lenses and adapted to be moved by said motion imparting members toward and away from said second optical set and to adjust focus; a fourth set of lenses for focusing the optical flow from the third set of lenses on said transducer members; the distance between said first and fourth sets of lenses being unchangeable.

In a preferred embodiment said second optical set comprises a positive meniscus lens and a negative doublet lens and has an overall negative focal length.

In a preferred embodiment said third set of lenses comprises a positive meniscus lens and a negative doublet lens and has an overall positive focal length.

In a preferred embodiment said first set of lenses comprises a negative doublet lens and a positive meniscus lens and has a positive focal length.

In a preferred embodiment said fourth set of lenses comprises a negative doublet lens a negative meniscus lens and a positive doublet lens and has a positive focal length.

In a preferred embodiment in each set of lenses the lenses have monotonically increasing or equal diameters, from the upstreammost lens to the downstreammost lens, relative to the input optical flow, or vice versa.

In a preferred embodiment it is provided a holder for each set of lenses having a plurality of seats, each designed to accommodate and hold a lens of the respective set of lenses in position.

In a preferred embodiment the seats of each holder which are designed to receive the upstreammost or downstreammost lens include a retaining shoulder which is designed to receive a surface portion of the lens abutting thereagainst. In a preferred embodiment it is provided a guide rods operable on all the holders; said guide rods mechanically and unremovably restraining therebetween the holder of the first set of lenses and the holder of the fourth set of lenses and slidably restraining therebetween and relative to the other holders, the holder of the second set of lenses and the holder of the third set of lenses.

In a preferred embodiment said motion imparting members include at least one first linear piezoelectric actuator operable on the second set of lenses and at least one second linear piezoelectric actuator operable on the third set of lenses; said first and second linear actuators being arranged in said housing downstream from the fourth set of lenses.

In a preferred embodiment the first and second linear actuators include respective pushers, operable on a holder of the second set of lenses and the third set of lenses respectively, to independently move the second and third set of lenses toward the first set of lenses.

In a preferred embodiment said pushers are mechanically disengaged from said holders; said pushers transferring forces to said holders which are only axially directed, relative to the axis along which the pushers extend.

In a preferred embodiment said motion imparting members include elastic members, operable on a holder of the second set of lenses and the third set of lenses, to independently push the second and third set of lenses toward the fourth set of lenses.

In a preferred embodiment said elastic members operate against said pushers of the first and second actuators; said pushers being adapted to be moved from and to said first set of lenses.

In a preferred embodiment said lighting members include LEDs located in a front end portion of the housing, to illuminate the environment outside the housing; said LEDs emitting an overall light flow ranging from 180 to 350 lumen.

In a preferred embodiment said lighting members include a light sensor located in a front end portion of the housing.

In a preferred embodiment a control and monitoring unit is in signal communication with an acquisition and processing system which is designed to implement a real-time image contrast analysis algorithm, via an intermediate image processing process based on contrast maximization.

In a preferred embodiment said intermediate process comprises the following steps:
- providing a k^{th} image detected by the transducer members,
- selecting a ROI and points distributed in said image;
- for each point, calculating the difference of its brightness from that of the neighboring pixels;
- summing such differences to generate a contrast index (Ck) of the k^{th} image;
- comparing such contrast index (Ck) with a reference value (Co),
- maximizing the contrast of said acquired image according to such comparison. In a preferred embodiment said step of comparing comprises the following additional steps:
- if said contrast index (Ck) is different from the reference value (Co), comparing said index (Ck) with a previous contrast index (Ck-1) of the previous interaction and
- if said contrast index (Ck) is increased as compared with said previous contrast index (Ck-1), i.e. Ck > Ck-1, then the direction of motion of said motion imparting members is toward increasing contrast values.

In a preferred embodiment said step of comparing comprises the following additional steps:
- if said contrast index (Ck) is different from the reference value (Co), comparing said index (Ck) with a previous contrast index (Ck-1) of the previous interaction and
- if said contrast index (Ck) is decreased as compared with said previous contrast index (Ck-1), i.e. Ck < Ck-1, then the direction of motion of said motion imparting members is toward decreasing contrast values.

In a preferred embodiment said acquisition and processing system processes a control for the autofocus motor that causes a displacement in the direction of the previous interaction by a value proportional to the difference between the reference value (Co) and said contrast index (Ck).

### BRIEF DESCRIPTION OF THE DRAWINGS

The features and advantages of the invention will appear from the following detailed description of one embodiment, which is illustrated without limitation in the annexed drawings, in which:
- Figure 1 shows a perspective view of an endoscope of the present invention;
- Figure 2 shows a perspective view of the endoscope of Figure 1, with certain parts omitted to better show other parts;
- Figure 3 shows a further perspective view of the endoscope of Figure 1, with certain parts omitted to better show other parts;
- Figure 4 shows a schematic view of certain details of the endoscope of Figure 1;
- Figures 5 to 8 show perspective views of further details of the endoscope of Figure 1;
- Figure 9 shows a block diagram of the electrical/electronic configuration of the endoscope of the present invention;
- Figure 10 shows a flow diagram of a target focusing function as implemented in the endoscope of the present invention;
- Figure 11 shows another flow diagram, suitable for controlling actuation of the optics according to the present invention;
- Figure 12 shows another flow diagram, suitable for controlling actuation illumination according to the present invention.

### DETAILED DESCRIPTION OF THE INVENTION:

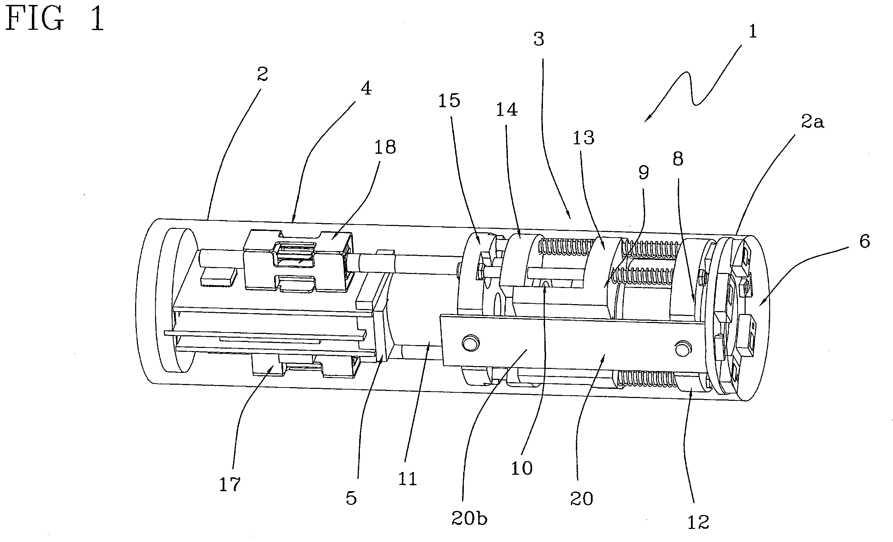

Referring now to Figure 1, numeral 1 generally designates an endoscope of the present invention.

The endoscope 1 comprises a substantially cylindrical housing 2, which is designed to be introduced in a body to be examined, an optical focusing and zooming device 3 contained in the housing, and motion imparting members 4 contained in the housing 2, operating on the optical device 3 and designed to move part of the optical device 3 for its focusing and zooming operation.

A control and monitoring unit 8 is also provided, which is configured to receive digital electric signals representative of optical zoom degrees and is operably connected to the motion imparting members 4 to actuate the latter according to the received optical zoom-representative signals.

The endoscope 1 also comprises transducer members 5 located in the housing 2 downstream from the optical device 3 and designed to convert an optical flow focused by the optical device 3 into an electric signal representative of optical zoom degrees.

In one embodiment, the transducer members 5 consist of a CMOS-based photodetector of photosensor.

For example, the CMOS is a Color CMOS commercially known as OV2720 manufactured by Omnivision, whose specifications are: full-hd 1080p and 30 fps. The pixel size is 1.4µm with OmniBSI technology (Backside Illumination) for improved low-light sensitivity, i.e. 680 mV/(lux·s).

It shall be noted that, as used in the present disclosure and accompanying claims, the terms "downstream" and "upstream" designate the relative position of two or more elements with respect to the direction of the optical flow (the electromagnetic radiation in the visible range) entering the endoscope 1. Particularly, what is placed upstream from an element is placed before such element, with respect to the optical flow that enters the endoscope; likewise what is placed downstream from an element is placed after such element with respect to the optical flow that enters the endoscope.

The endoscope 1 further comprises lighting members 6 comprising at least one light source 7 located in the housing 2.

The housing 2 contains the above mentioned parts of the endoscope, which are sealed within the housing 2. Thus, the endoscope 1 may be sterilized between applications by placing the housing 2 (and all the parts therein) in an autoclave. The housing 2 is also preferably made of a metal material, e.g. titanium.

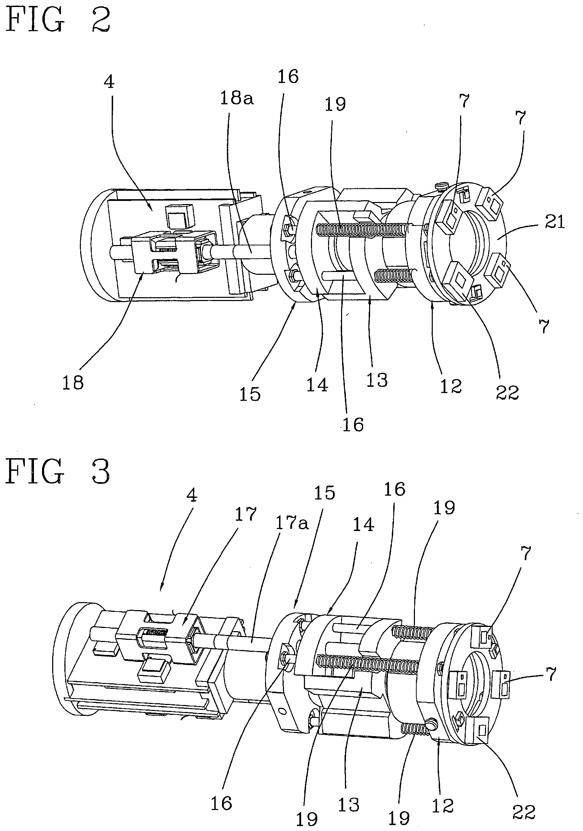

The optical device 3, which is generally shown in Figure 1 and illustrated in greater detail in Figures 2 to 8, comprises a first set of lenses 8, which is designed to cause an input optical flow to converge toward a second set of lenses 9. The second set of lenses 9 is located downstream from the first set of lenses 8 and is adapted to be moved by the motion imparting means 4 toward and away from the first optical set 8 and to change the overall optical zoom. The optical device 3 also comprises a third set of lenses 10 located downstream from the second set of lenses 9 and also adapted to be moved by the motion imparting means 4 toward and away from the second optical set 9 and to provide focus adjustment. A fourth set of lenses 11 is designed to focus the optical flow from the third set of lenses 10 on the transducer members 5. The distance between the first 8 and the fourth 11 set of lenses is unchangeable, whereas the second 9 and third 10 sets o lenses may be moved toward and away from each other and toward and away from the first and fourth sets of lenses. The optical device 3 with the above mentioned four sets of lenses allows use of the endoscope 1 in applications in which the distance from the target ranges from about 30 mm to 300 mm, with an angle of view ranging from about 40° to 70°, preferably from 50° to 60°, and with a depth of field of at least 1.5 cm over the whole range of distances. Thus, this optical device 3 with focusing and zooming features allows the surgeon, for example, to explore the abdominal cavity without physically moving the endoscope toward the target, thereby avoiding any undesired contact that might damage patient tissues.

Particularly, the first set of lenses 8 has the purpose of focusing the image on the second set of lenses 9 at a wide aperture angle. The second set of lenses is movable, and is moved by the motion imparting members 4, its purpose being to change the focal length of the whole optical device 3 to change the relevant aperture angle without changing the height of the image. Since this causes the virtual image plane (i.e. the plane on which the optical flow is focused) to move, which means that the more the target is magnified (the aperture angle decreases), the closer the virtual image plane is moved toward the fourth set of lenses 11, the third set of lenses 10 is movable (by the action of the motion imparting means 4) to cause the virtual image plane to coincide with the physical image plane of the transducer members. It shall be noted that the third set of lenses 10 moves the virtual image plane without changing the magnification factor. Furthermore, as described above, the third set of lenses 10 can reposition the virtual image plane in its proper position by changing the optical zoom due to the action of the motion imparting members 4. As mentioned above, the fourth set of lenses 11 is stationary and has the purpose of focusing the image on the transducer members.

The operation of the motion imparting members 4 affords proper automatic focusing (i.e. with no corrective action to be taken by the user).

This target distance changing operation, followed by focus adjustment (i.e. to properly position the virtual image) is accomplished by a closed-loop control as described hereinbefore.

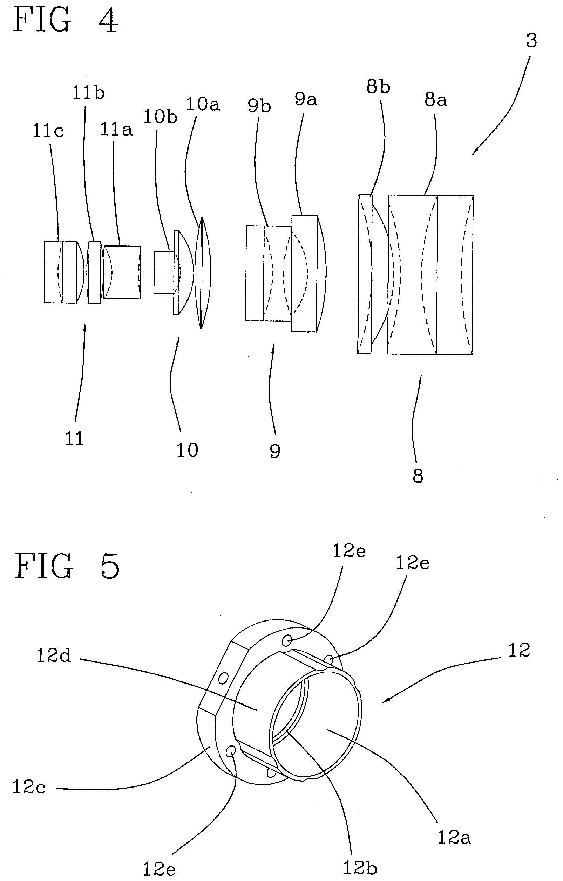

As shown in Figure 4, the first set of lenses 8 is composed of three lenses and comprises a negative doublet lens 8a and a positive meniscus lens 8b. The first set of lenses 8 has a positive focal length. The second set of lenses 9 is composed of three lenses and comprises a positive meniscus lens 9a and a negative doublet lens 9b. The second set of lenses 9 has a negative focal length. The third set of lenses 10 is composed of three lenses and comprises a positive meniscus lens 10a and a positive doublet lens 10b. The third set of lenses 10 has a positive focal length. The fourth set of lenses 11 is composed of four lenses and comprises a negative doublet lens 11a, a negative meniscus lens 11b and a positive doublet lens 11c. The fourth set of lenses 11 has a positive focal length. The lenses of each of the above sets of lenses are in orderly succession from upstream to downstream from the optical flow entering the optical device 3.

As shown in Figure 4, in each set of lenses the lenses have monotonically decreasing or equal diameters, from the upstreammost lens to the downstreammost lens, with respect to the optical flow that enters such set, or vice versa. In other words, the lenses of each set of lenses are disposed in succession such that lenses having equal or decreasing diameters are found from upstream to downstream (or vice versa). This feature allows the lenses to be placed in their operating position by inserting the lenses of each set one after another, from the largest lens (i.e. the one with the largest diameter) to the smallest lens. This lens positioning arrangement is very easy and simple and allows insertion of the lenses of each set in the proper functional order.

In this respect, the endoscope 1 comprises a holder 12, 13, 14, 15 for each set of lenses. Each holder comprises a plurality of seats 12a, 13a, 14a, 15a, each designed to accommodate and hold a lens of the respective set of lenses in position. Particularly, the seats of each holder which are designed to receive the upstreammost or downstreammost lens, with respect to the optical flow entering the set, include a retaining shoulder 12b, 13b, 14b, 15b which is designed to receive a surface portion of the lens abutting thereagainst (see Figures 5 to 8).

Preferably, as shown in Figure 5, the holder 12 for the first set of lenses 8 (hereinafter referred to as first holder) comprises an annular flange 12c in which the seat 12b for holding the upstreammost lens of the first set of lenses is formed. A cylindrical sleeve 12d for holding the other lenses of the first set of lenses 8 extends from the flange 12c. It shall be noted that all the lenses of the first set have the same diameter. The doublets 12a of the first set of lenses 8, as well as the doublets of the other sets of lenses are joined together by highly transparent optical cement. The flange 12c also comprises a plurality of holes 12e with axes parallel to the longitudinal axis of the above mentioned sleeve 12d and designed for connecting the holders together (as detailed hereinbelow).

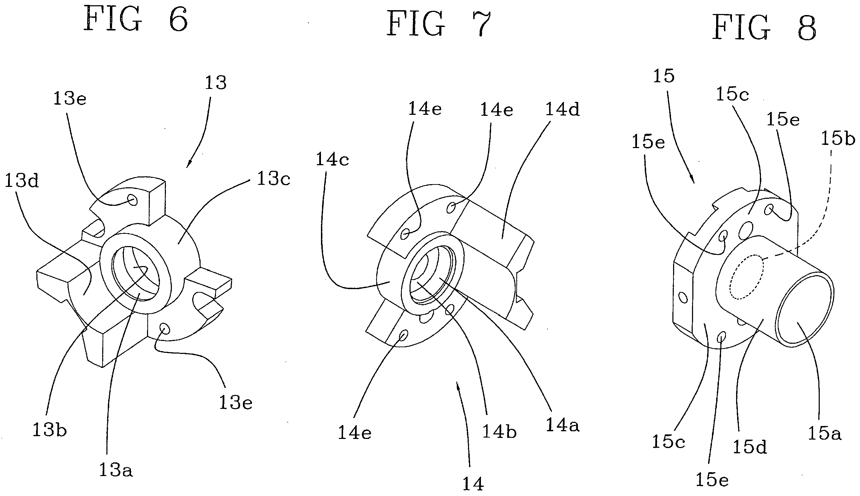

As shown in Figure 6, the holder 13 for the second set of lenses 9 (hereinafter referred to as second holder) comprises a flange 13c in which the seat 13b for holding the downstreammost lens of the second set of lenses 9 is formed. The other lenses of the second set of lenses 9 are also held within the flange 13c. It shall be noted that the downsterammost lens of the second set of lenses 9 (i.e. the lens that will be held in the seat 13b) has a smaller diameter than the upstreammost lens. The lens intervening between the two has the same diameter as the downstreammost lens. A tailpiece 13d is connected to the flange 13c, and is designed to retain a part of a movement sensor 20 (better described below). The flange 13c further comprises a plurality of holes 13e designed for connecting the holders together (as detailed hereinbelow).

As shown in Figure 7, the holder 14 for the third set of lenses 10 (hereinafter referred to as third holder) comprises a flange 14c in which the seat 14b for holding the downstreammost lens of the second set of lenses 8 is formed. The other lenses of the third set of lenses 10 are also held within the flange 14c. It shall be noted that the downsterammost lens of the third set of lenses 10 (i.e. the lens that will be held in the seat 14b) has a smaller diameter than the upstreammost lens. The lens intervening between the two has a diameter that is intermediate the ones of the upstream lens and the downstream lens. A tailpiece 14d is connected to the flange 14c, and is designed to retain the above mentioned part of the movement sensor 20. The flange 14c further comprises a plurality of holes 14e designed for connecting the holders together (as detailed hereinbelow).

As shown in Figure 8, the holder 15 for the fourth set of lenses 11 (hereinafter referred to as fourth holder) comprises an annular flange 15c in which the seat 15b for holding the downstreammost lens of the fourth set of lenses 11 is formed. A cylindrical sleeve 15d for holding the other lenses of the fourth set of lenses 11 extends from the flange 15c. It shall be noted that the first three lenses of the fourth set 11 have the same diameter, whereas the upstreammost lens has a smaller diameter than those first three lenses. The flange 15c also comprises a plurality of holes 15e with axes parallel to the longitudinal axis of the above mentioned sleeve 15d and designed for connecting the holders together (as detailed hereinbelow).

Preferably, each holder has a monolithic construction, i.e. is formed of one piece. The holders 12, 13, 14, 15 are preferably made of a diamagnetic material, e.g. the 6061 aluminum alloy, i.e. an aluminum alloy composed of 0.6% silicon, 0.25 copper, 1% magnesium and 0.2 chromium.

As mentioned above, the holders are joined together. Particularly, guide rods 16 (see Figures 2 and 3) are provided to join the first holder 12 and the fourth holder 15 together, while maintaining them at a preset distance from each other. The guide rods 16 are engaged in the above mentioned holes 12e and 15e of the flanges of the first 12 and fourth 15 holders. The guide rods 16 are also slidingly engaged in the holes 13e and 14e of the flanges of the second 13 and third 14 holders. Thus, the second 9 and third 10 sets of lenses may slide from and toward the first 8 and fourth 11 sets of lenses. Preferably, the guide rods 16 are made of polytetrafluoroethylene (PTFE), to reduce friction between the rods and the second and third holders.

The motion imparting members 4 include at least one first linear piezoelectric actuator 17 operable on the second set of lenses 9 and at least one second linear piezoelectric actuator 18 operable on the third set of lenses 10. The first 17 and second 18 linear actuators are arranged in the holder 2 downstream from the fourth set of lenses 11. Particularly, the first 17 and second 18 linear piezoelectric actuators are operable on the second 14 and third 14 holders respectively. The first 17 and second 18 linear actuators include respective pushers 17a, 18a, operable on the second holder 13 (preferably on its flange 13c) and on the third holder 14 (preferably on its flange 14c) respectively, to independently move the second and third sets of lenses toward the first set of lenses. In other words, the pushers 17a, 18a can push the holders on which they are operable toward the first set of lenses 8 and cannot pull them toward the fourth set of lenses 11. For this purpose, the pushers 17a, 18a are mechanically disengaged from the second 13 and third 14 holders and transmit forces to the latter, which are only directed along the longitudinal axis of the pushers. In other words, the pushers 17a, 18a are coupled to their respective holders such that the pushers and the holders only exchange axial actions, with no shear forces or torques being ever transmitted therebetween. In order that the second 13 and third 14 holders may move in a controlled manner toward the fourth set of lenses 11, the motion imparting members 4 comprise elastic members 19, operable between the first holder 12 and the second 13 and third 14 holders respectively, to independently push the second 9 and third 10 sets of lenses toward the fourth set of lenses 11. These elastic members 19 operate against the pushers 17a, 18a. Therefore, when the pushers 17a, 18a move toward the first set of lenses 8, the pushers exert a greater axial force than the elastic members 19, thereby moving the second 13 and/or third 14 holders toward the first set of lenses 8. When the pushers 17a, 18a move toward the fourth set of lenses 11, the elastic members 19 push the second 13 and/or third 14 holders toward the fourth set of lenses 11, thereby keeping them in contact with the pushers. The elastic members 19 are guided by the above mentioned guide rods 16 and preferably consist of linear springs fitted on the guide rods 16, preferably made of stainless steel.

Preferably, the first 17 and second 18 linear piezoelectric actuators are of the type in which the pusher is a threaded bolt, which is pushed to and fro into a matingly threaded nut, composed of four piezoelectric plates stimulated by voltage signals modulated at appropriately phase-shifted and inverted sonic frequencies. This kind of linear piezoelectric actuator is known with the trade name of "Squiggle".

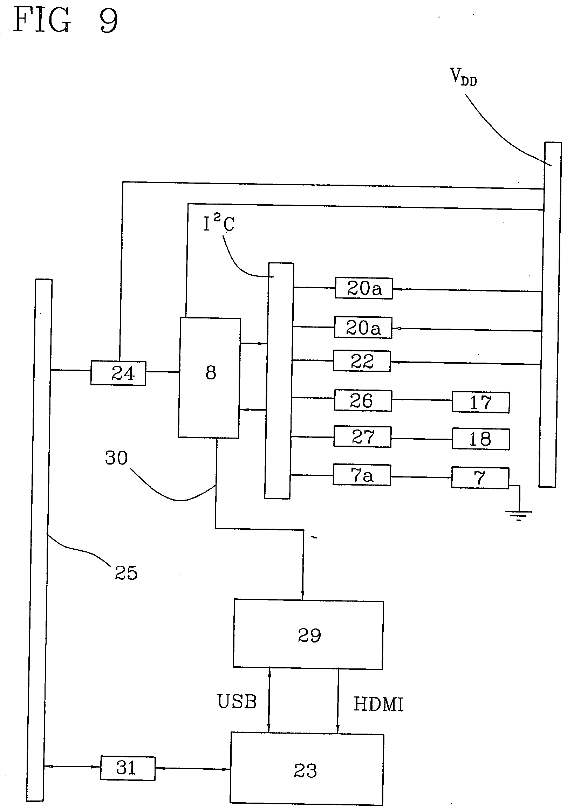

The endoscope 1 comprises the above mentioned movement sensors 20 (see Figure 1). These sensors 20 have the purpose of determining the relative position assumed by the second 13 and third 14 holders with respect to a fixed reference in the housing 2. Preferably, these sensors 20 are two Hall effect encoders 20a (as schematically shown in Figure 9) each being operable on the second 13 or third 14 holder. Particularly, each encoder 20a comprises a magnet (not shown) which is integral with the second 13 or third 14 holder, and is preferably integral with their tailpieces 13d and 14d, and a transducer 20b (that can detect magnetic flow changes) which is integral with the housing 2.

The light source 7 of the lighting members 6 comprises LEDs located in a front end portion 2a of the housing 2, to illuminate the environment outside the housing. These LEDs 7 are arranged over an annulus 21 placed upstream from the first set of lenses 8. The annulus 21 integrates the driver 7a for the LEDs 7. The LEDs emit a total luminous flux ranging from 180 to 350 lumens, preferably of about 225 lumens. The lighting members 6 include a light sensor 22 located in a front end portion 2a of the housing 2. Preferably, the light sensor 22 is also placed on the annulus 21, the latter integrating the driver of the light sensor 22.

Referring now to Figure 9, there is shown a block diagram of the electrical/electronic configuration of the endoscope 1, namely when the endoscope 1 is in signal communication with:
- an external computer unit 23 having a user interface therein (not shown) which allows the operator, in one aspect, to control the optical and digital zoom, the illumination and certain features of the transducer members 5;
- an acquisition and processing system 29, which is in turn in signal communication with the external computer unit 23.

As shown by the block diagram, the control and monitoring unit 8 of the endoscope 1 is powered by a supply voltage Vdd and is interfaced through a voltage level adapter 24 with a CAN bus 25 to send/receive the control signals from the external unit 23 having the user interface thereon.

The control and monitoring unit 8 is in signal communication with the various devices that form the endoscope 1, such as the piezoelectric actuators 17, 18, the transducer members 5, the light source 7, the encoders 20a, using the digital protocol known as I²C.

In one aspect, the control and monitoring unit 8 consists of a microcontroller µC, with one or more firmware programs possibly stored in the memory of such microcontroller, for:
- encoding and decoding CAN data packets,
- encoding and decoding internal I²C-based internal communications, to manage interoperability of the various devices, e.g. to control the piezoelectric actuators, to track the position of the actuators by the encoders, to control the lighting LEDs, to read the general environmental luminosity, to control the CMOS for adjustment of the digital zoom and image brightness,
- generating a clock signal for the CMOS sensor.

In one aspect, the microcontroller is in signal communication with two drivers 26, 27, to control and monitor the two piezoelectric actuators 17, 18 respectively.

In one aspect, the microcontroller µC is in I²C-based signal communication, through an appropriate driver 28, with the transducer members 5, i.e. the CMOS light sensor, which-is powered by the supply voltage Vdd.

In one aspect, the microcontroller µC is in I²C-based signal communication, through an appropriate driver 28, with the light source 7 of the lighting members 6 which, in the preferred embodiment.

In one embodiment, the light source 7 is composed of LEDs, and hence the driver 7a is a LED driver.

In one aspect, as shown in the block diagram, the control and monitoring unit 8 of the endoscope 1 interfaces with the acquisition and processing system 29, for example, through an electric signal 30 of the Digital Video Port (DVP) type.

The acquisition and processing system 29 comprises a board having a Digital Signal Processor (DSP) which is configured for implementation of:
- a first algorithm, which is known to the skilled person and will not be described herein, that can:
   - decode the DVP electric signal 30 at the output of the transducers 5 (i.e. the CMOS sensor) via the microcontroller of the control and monitoring unit 8 and
   - encode it into the High Definition Multimedia Interface (HDMI) standard, which allows it to display the image on a screen, e.g. a high definition HD screen of the computer 23;
- a second algorithm for real-time image contrast analysis, which is adapted to drive the autofocus mechanism of the viewing apparatus;
- a third algorithm for digital image stabilization, because the position of the camera cannot be fixed.

In one embodiment, the board with the DSP 29 is in signal communication with the computer 25 via a USB interface and the computer 23 interfaces with the CAN bus 25 via a CANUSB dongle 31.

In one embodiment, the endoscope 1 is configured to magnify a target located at a given distance. Particularly, as distance changes, the virtual focusing plane moves. Therefore, as the target being viewed by the endoscope comes closer, the distance from the virtual focusing plane increases.

The image may be refocused by changing the position of the third set of lenses 10.

In one embodiment, this target distance changing operation, followed by focus adjustment, may be automatically accomplished by a closed-loop control.

For this purpose, focus is adjusted by the optical device 3 using a mechanical compensation feature (MC), in which the operating distance of the target from the optical device 3 is fixed and the user can magnify the target.

For this purpose, the second set of lenses 9 performs magnification and the third set of lenses 10 compensates for the movement of the image plane.

The relative positions of the second and third sets of lenses 9, 10 are defined by given curves, that are described by look-up tables stored in the memory of the microcontroller µC.

These look-up tables contain the positions of the second and third sets of lenses 9, 10 according to the magnification factor desired by the user.

This process is used, for instance, when the user positions the endoscope and wants to see a greater amount of details (i.e. increase magnification) or obtain a wider field of view (i.e. decrease magnification).

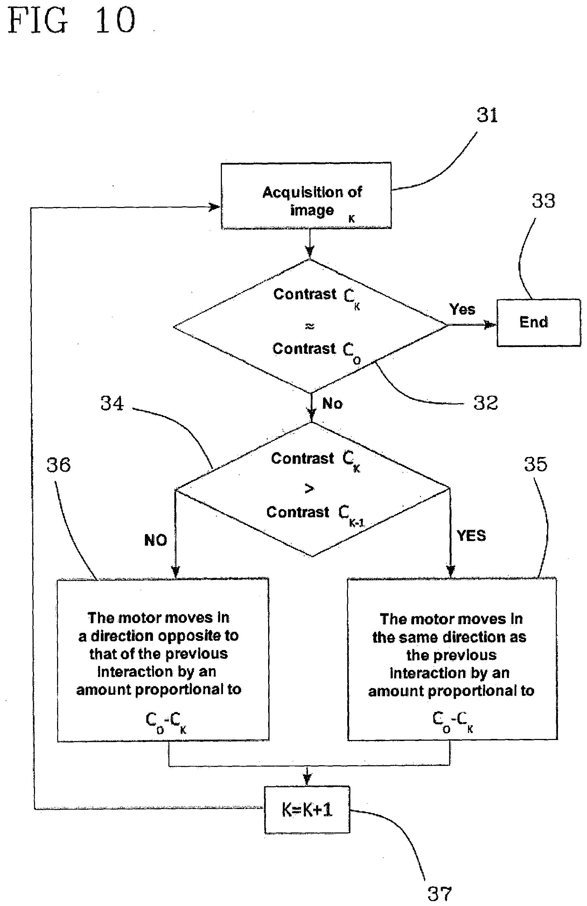

In a further aspect, the target distance changing operation, followed by focus adjustment, may be accomplished by an autofocus (AF) feature that, referring to Figure 10, is implemented by means of the second algorithm for real-time image contrast analysis.

This second algorithm is adapted to drive the autofocus mechanism of the various optical sets 8, 9, 10 and 11, and allow the target to be focused for a preset magnification factor, irrespective of target distance.

Particularly, since the target distance is not defined, once the endoscope 1 has been positioned the target is focused by an intermediate image processing routine, based on contrast maximization performed in the DSP processing board 29.

Therefore, in one aspect, contrast is the function to be maximized by such second algorithm.

For this reason, the algorithm considers the k^{th} image detected by the transducer members 5 (one color component, e.g. green, will be simply needed), selects one Region of Interest (ROI) and some pixels therein and calculates for each pixel the difference between its brightness and that of the neighboring pixels. The sum of these differences provides a contrast index Ck for the k^{th} image, block 31 of the flow diagram of Figure 10.

This contrast index Ck is compared with an optimal reference value Co, block 32.

If the two values Ck and Co match, branch YES of block 32, then the image contrast is already at its optimal value, block 33 otherwise, branch NO of block 32, Ck is compared with Ck-1 of the previous interaction, block 34.

If contrast has increased (Ck> Ck-1), branch YES of the block 34, then the direction of movement is toward increasing contrast values, block 35.

The DSP processes a control for the autofocus motor that causes a movement, still in the direction of the previous interaction by a value proportional to the difference Co-Ck.

The smaller this difference the greater the contrast Ck, and the closer the support to the position in which the image is focused.

The direction of movement will be reversed, block 36, if contrast Ck has worsened (Ck< Ck-1), branch NO of block 34.

The operation is repeated until the image contrast is equal to the optimal contrast, block 37.

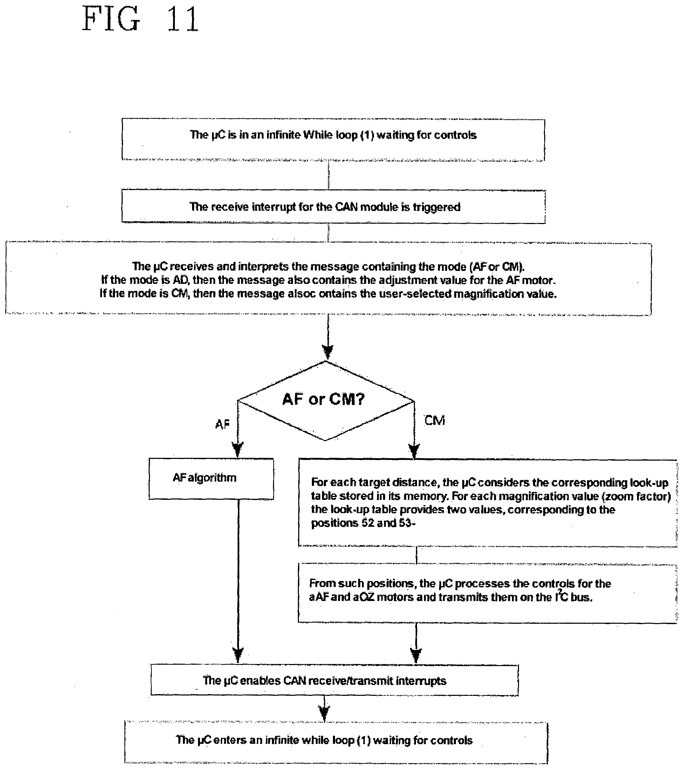

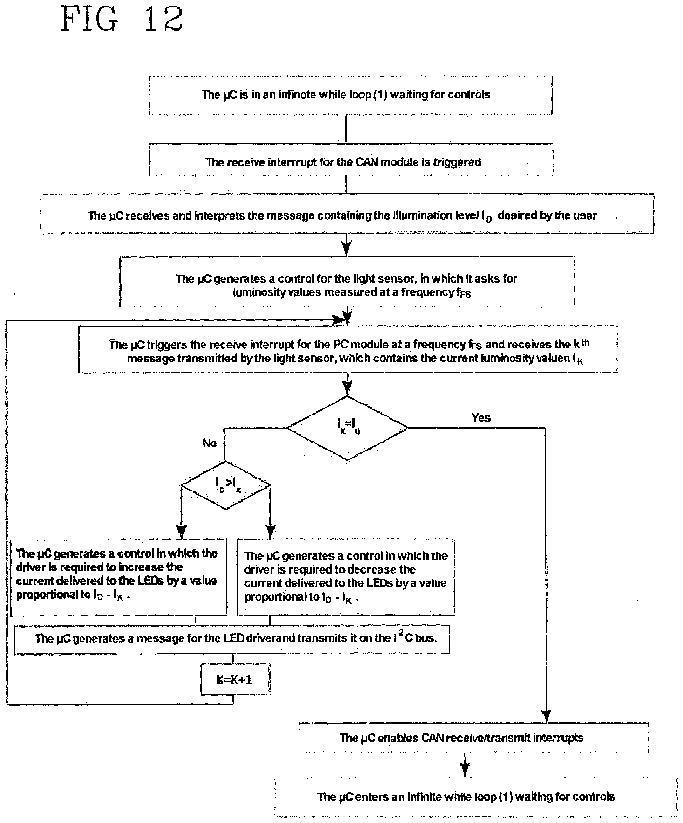

Concerning the firmware stored in the memory of the µC and implemented thereby, in one aspect:
- the firmware for controlling the transducer members 5, i.e. the CMOS light sensor, requires the user to adjust the digital zoom and/or the overall brightness of the image at the output of the CMOS, using the graphics interface on the external unit 23, according to the digital zoom or image brightness values desired by the user;
- the firmware for controlling optics operation, also referring to Figure 11, allows the default user to be, for example, in Autofocus mode AF and to switch to Manual mode CM. If the user is in the AF mode, then the microcontroller µC either executes the AF algorithm (as described above) or controls the motors 17, 18 to move the respective holders 13, 14 to their appropriate positions;
- the firmware for controlling illumination, also referring to Figure 12, requires the microcontroller µC to read a luminosity level in the environment in which the endoscope 1 is placed, using the transducer members 5, i.e. the CMOS light sensor. According to the user-defined luminosity value, the firmware processes and sends a control for the driver 7a of the light source 7, i.e. the LEDs, to adjust the current delivered to the LEDs.

Those skilled in the art will obviously appreciate that a number of changes and variants may be made to the above described optical instrument and viewing system, to meet incidental and specific needs, without departure from the inventive scope, as defined in the following claims.

## Claims

1. An endoscope comprising:
- a substantially cylindrical housing (2), which is designed to be introduced into a body to be examined;
- an optical focusing and zooming device (3) contained in said housing (2);
- motion imparting members (4) contained in said housing (2), operating on said optical device (3) and designed to move part of said optical device (3) for its focusing and zooming operation;
- transducer members (5) located in said housing (2) downstream from said optical device (3) and designed to convert an optical flow focused by said optical device (3) into an electric signal;
- lighting members (6) comprising at least one light source (7) located in the housing (2);
- a control and monitoring unit (8), which is designed to receive signals representative of optical zoom degrees and is operably connected to said motion imparting members (4) to actuate said motion imparting members (4) according to the received optical zoom-representative signals;
- said optical device (3) comprising a first set of lenses (8) designed to cause an input optical flow to converge toward a second set of lenses (9), said second set of lenses (9) being located downstream from said first set of lenses (8) and being adapted to be moved by said motion imparting means (4) toward and away from said first optical set (8) and to change the overall optical zoom; a third set of lenses (10) located downstream from said second set of lenses (9) and adapted to be moved by said motion imparting members (4) toward and away from said second optical set (9) and to adjust focus; **characterized in that** the optical device further comprises a fourth set of lenses (11) for focusing the optical flow from the third set of lenses (10) on said transducer members (5); the distance between said first (8) and fourth (11) sets of lenses being unchangeable.

2. An endoscope as claimed in claim 1, wherein said second optical set (9) comprises a positive meniscus lens (9a) and a negative doublet lens (9b) and has an overall negative focal length.

3. An endoscope as claimed in claim 1 or 2, wherein said third set of lenses (10) comprises a positive meniscus lens (10a) and a negative doublet lens (10b) and has an overall positive focal length.

4. An endoscope as claimed in any preceding claim, wherein said first set of lenses (8) comprises a negative doublet lens (8a) and a positive meniscus lens (8b) and has a positive focal length.

5. An endoscope as claimed in any preceding claim, wherein said fourth set of lenses (11) comprises a negative doublet lens (11a) a negative meniscus lens (11b) and a positive doublet lens (11c) and has a positive focal length.

6. An endoscope as claimed in any preceding claim wherein, in each set of lenses (8, 9, 10, 11), the lenses have monotonically increasing or equal diameters, from the upstreammost lens to the downstreammost lens, relative to the input optical flow, or vice versa.

7. An endoscope as claimed in any preceding claim, comprising a holder (12, 13, 14, 15) for each set of lenses (8, 9, 10, 11) having a plurality of seats (12a, 13a, 14a, 15a), each designed to accommodate and hold a lens of the respective set of lenses in position.

8. An endoscope as claimed in claim 7, wherein the seats (12a, 13a, 14a, 15a) of each holder (12, 13, 14, 15) which are designed to receive the upstreammost or downstreammost lens include a retaining shoulder (12b, 13b, 14b, 15b) which is designed to receive a surface portion of the lens abutting thereagainst.

9. An endoscope as claimed in claim 7, comprising guide rods (16) operable on all the holders (12, 13, 14, 15); said guide rods (16) mechanically and unremovably restraining therebetween the holder (12) of the first set of lenses (8) and the holder (15) of the fourth set of lenses (11) and slidably restraining therebetween and relative to the other holders, the holder (13) of the second set of lenses (9) and the holder (14) of the third set of lenses (10).

10. An endoscope as claimed in any preceding claim, wherein said motion imparting members (4) include at least one first linear piezoelectric actuator (17) operable on the second set of lenses (9) and at least one second linear piezoelectric actuator (18) operable on the third set of lenses (9); said first (17) and second (18) linear actuators being arranged in said housing (2) downstream from the fourth set of lenses (11).

11. An endoscope as claimed in claim 10, wherein the first (17) and second (18) linear actuators include respective pushers (17a, 18a), operable on a holder (13, 14) of the second set of lenses (9) and the third set of lenses (10) respectively, to independently move the second (9) and third (10) set of lenses toward the first set of lenses (8).

12. An endoscope as claimed in claim 11, wherein said pushers (17a, 18a) are mechanically disengaged from said holders (13, 14); said pushers (17a, 18a) transferring forces to said holders (13, 14) which are only axially directed, relative to the axis along which the pushers (17a, 18a) extend.

13. An endoscope as claimed in claim 11 or 12, wherein said motion imparting members (4) include elastic members (19), operable on a holder (13, 14) of the second set of lenses (9) and the third set of lenses (10), to independently push the second (9) and third (10) set of lenses toward the fourth set of lenses (11).

14. An endoscope as claimed in claim 1, wherein the control and monitoring unit (8) is in signal communication with an acquisition and processing system (29) which is designed to implement a real-time image contrast analysis algorithm, via an intermediate image processing process based on contrast maximization.

15. An endoscope as claimed in claim 14, wherein said intermediate process comprises the following steps:
- providing a k^{th} image detected by the transducer members,
- selecting a ROI and points distributed in said image;
- for each point, calculating the difference of its brightness from that of the neighboring pixels;
- summing such differences to generate a contrast index (Ck) of the k^{th} image;
- comparing such contrast index (Ck) with a reference value (Co),
- maximizing the contrast of said acquired image according to such comparison.

## Patentansprüche

1. Endoskop, umfassend:
- ein im Wesentlichen zylindrisches Gehäuse (2), das dazu bestimmt ist, in einen zu untersuchenden Körper eingeführt zu werden;
- eine optische Fokussier- und Zoomeinrichtung (3), die in dem Gehäuse (2) untergebracht ist;
- Bewegungsvermittlungselemente (4), die in dem Gehäuse (2) untergebracht sind, auf die optische Einrichtung (3) wirken und dazu bestimmt sind, einen Teil der optischen Einrichtung (3) für deren Fokussier- und Zoomvorgang zu bewegen;
- Wandlerelemente (5), die in dem Gehäuse (2) stromabwärts der optischen Einrichtung (3) angeordnet und dazu bestimmt sind, einen von der optischen Einrichtung (3) gebündelten optischen Fluss in ein elektrisches Signal umzuwandeln;
- Beleuchtungselemente (6), umfassend wenigstens eine in dem Gehäuse (2) angeordnete Lichtquelle (7);
- eine Steuer- und Überwachungseinheit (8), die dazu bestimmt ist, Signale zu empfangen, die repräsentativ für optische Zoomgrade sind, und die mit den Bewegungsvermittlungselementen (4) wirkverbunden ist, um die Bewegungsvermittlungselemente (4) gemäß den empfangenen optischen, für das Zoom repräsentativen Signalen zu betätigen;
- wobei die optische Einrichtung (3) einen ersten Satz von Linsen (8) umfasst, die dazu bestimmt sind, einen optischen Eingangsfluss dazu zu veranlassen, auf einen zweiten Satz von Linsen (9) hin zu konvergieren, wobei der zweite Satz von Linsen (9) stromabwärts des ersten Satzes von Linsen (8) angeordnet und dazu geeignet ist, von den Bewegungsvermittlungselementen (4) zu und weg von dem ersten optischen Satz (8) bewegt zu werden und den optischen Gesamtzoom zu ändern; einen dritten Satz von Linsen (10), der stromabwärts des zweiten Satzes von Linsen (9) angeordnet und dazu geeignet ist, von den Bewegungsvermittlungselementen (4) zu und weg von dem zweiten optischen Satz (9) bewegt zu werden und sich scharfeinzustellen; **dadurch gekennzeichnet, dass** die optische Einrichtung ferner einen vierten Satz von Linsen (11) zum Fokussieren des optischen Flusses von dem dritten Satz von Linsen (10) auf die Wandlerelemente (5) umfasst; wobei der Abstand zwischen dem ersten (8) und vierten (11) Satz von Linsen unveränderbar ist.

2. Endoskop nach Anspruch 1, wobei der zweite optische Satz (9) eine positive Meniskuslinse (9a) und eine negative Doppellinse (9b) umfasst und eine negative Gesamtbrennweite aufweist.

3. Endoskop nach Anspruch 1 oder 2, wobei der dritte Satz von Linsen (10) eine positive Meniskuslinse (10a) und eine negative Doppellinse (10b) umfasst und eine positive Gesamtbrennweite aufweist.

4. Endoskop nach einem der vorstehenden Ansprüche, wobei der erste Satz von Linsen (8) eine negative Doppellinse (8a) und eine positive Meniskuslinse (8b) umfasst und eine positive Brennweite aufweist.

5. Endoskop nach einem der vorstehenden Ansprüche, wobei der vierte Satz von Linsen (11) eine negative Doppellinse (11a), eine negative Meniskuslinse (11b) und eine positive Doppellinse (11c) umfasst und eine positive Brennweite aufweist.

6. Endoskop nach einem der vorstehenden Ansprüche, wobei in jedem Satz von Linsen (8, 9, 10, 11) die Linsen - von der am meisten stromaufwärts gelegenen Linse bis zur am meisten stromabwärts gelegenen Linse - relativ zum optischen Eingangsfluss monoton steigende oder gleiche Durchmesser, oder umgekehrt, aufweisen.

7. Endoskop nach einem der vorstehenden Ansprüche, umfassend einen Halter (12, 13, 14, 15) für jeden Satz von Linsen (8, 9, 10, 11) mit einer Mehrzahl von Sitzen (12a, 13a, 14a, 15a), welche jeweils dazu bestimmt sind, eine Linse des jeweiligen Satzes von Linsen aufzunehmen und in Position zu halten.

8. Endoskop nach Anspruch 7, wobei die Sitze (12a, 13a, 14a, 15a) eines jeden Halters (12, 13, 14, 15), die dazu bestimmt sind, die am meisten stromaufwärts oder stromabwärts gelegene Linse aufzunehmen, einen Halteabsatz (12b, 13b, 14b, 15b) aufweisen, der dazu bestimmt ist, einen daran in Anstoß gehenden Oberflächenabschnitt der Linse aufzunehmen.

9. Endoskop nach Anspruch 7, umfassend Führungsstäbe (16), die auf allen Haltern (12, 13, 14, 15) betätigbar sind; wobei die Führungsstäbe (16) den Halter (12) des ersten Satzes von Linsen (8) und den Halter (15) des vierten Satzes von Linsen (11) dazwischen mechanisch und unlösbar zurückhalten und den Halter (13) des zweiten Satzes von Linsen (9) und den Halter (14) des dritten Satzes von Linsen (10) relativ zu den anderen Haltern und dazwischen verschiebbar zurückhalten.

10. Endoskop nach einem der vorstehenden Ansprüche, wobei die Bewegungsvermittlungselemente (4) wenigstens ein erstes lineares piezoelektrisches Betätigungselement (17) aufweisen, das auf dem zweiten Satz von Linsen (9) betätigbar ist, und wenigstens ein zweites lineares piezoelektrisches Betätigungselement (18), das auf dem dritten Satz von Linsen (9) betätigbar ist; wobei das erste (17) und das zweite (18) lineare Betätigungselement in dem Gehäuse (2) stromabwärts des vierten Satzes von Linsen (11) angeordnet sind.

11. Endoskop nach Anspruch 10, wobei das erste (17) und das zweite (18) lineare Betätigungselement jeweilige Schubelemente (17a, 18a) aufweisen, die jeweils auf einem Halter (13, 14) des zweiten Satzes von Linsen (9) und des dritten Satzes von Linsen (10) betätigbar sind, um den zweiten (9) und den dritten (10) Satz von Linsen unabhängig zum ersten Satz von Linsen (8) zu bewegen.

12. Endoskop nach Anspruch 11, wobei die Schubelemente (17a, 18a) mechanisch außer Eingriff mit den Haltern (13, 14) sind; wobei die Schubelemente (17a, 18a) Kräfte auf die nur axial ausgerichteten Halter (13, 14) übertragen, relativ zu der Achse, entlang welcher sich die Schubelemente (17a, 18a) erstrecken.

13. Endoskop nach Anspruch 11 oder 12, wobei die Bewegungsvermittlungselemente (4) elastische Elemente (19) aufweisen, die auf einem Halter (13, 14) des zweiten Satzes von Linsen (9) und des dritten Satzes von Linsen (10) betätigbar sind, um den zweiten (9) und den dritten (10) Satz von Linsen unabhängig zum vierten Satz von Linsen (11) zu verschieben.

14. Endoskop nach Anspruch 1, wobei die Steuer- und Überwachungseinheit (8) in Signalverbindung mit einem Erfassungs- und Verarbeitungssystem (29) steht, das dazu bestimmt ist, einen Echtzeit-Bildkontrast-Analysealgorithmus über ein Bildverarbeitungszwischenverfahren basierend auf Kontrastmaximierung zu implementieren.

15. Endoskop nach Anspruch 14, wobei das Zwischenverfahren folgende Schritte umfasst:
- Bereitstellen eines von den Wandlerelementen erfassten k-ten Bildes,
- Auswählen von über das Bild verteilten interessierenden Bereichen (ROI) und Stellen;
- für jede Stelle Berechnen des Unterschiedes zwischen ihrer Helligkeit und der der benachbarten Pixel;
- Addieren solcher Unterschiede, um einen Kontrastindex (Ck) des k-ten Bildes zu erzeugen;
- Vergleichen eines solchen Kontrastindexes (Ck) mit einem Referenzwert (Co),
- Maximieren des Kontrasts des erfassten Bildes gemäß eines solchen Vergleichs.

## Revendications

1. Endoscope comprenant :
- un boîtier sensiblement cylindrique (2), qui est conçu pour être introduit dans un corps à examiner ;
- un dispositif optique de focalisation et de zoom (3) contenus dans ledit boîtier (2) ;
- des éléments de communication de mouvement (4) contenus dans ledit boîtier (2), fonctionnant sur ledit dispositif optique (3) et conçus pour déplacer une partie dudit dispositif optique (3) pour son opération de focalisation et de zoom ;
- des éléments transducteurs (5) situés dans ledit boîtier (2) en aval dudit dispositif optique (3) et conçus pour convertir un flux optique focalisé par ledit dispositif optique (3) en un signal électrique ;
- des éléments d'éclairage (6) comprenant au moins une source de lumière (7) située dans le boîtier (2) ;
- une unité de commande et de surveillance (8), qui est conçue pour recevoir des signaux représentatifs de degrés de zoom optique et connectée fonctionnellement auxdits éléments de communication de mouvement (4) pour actionner lesdits éléments de communication de mouvement (4) en fonction des signaux reçus représentatifs du zoom optique ;
- ledit dispositif optique (3) comprenant un premier ensemble de lentilles (8) conçu pour amener un flux optique d'entrée à converger vers un deuxième ensemble de lentilles (9), ledit deuxième ensemble de lentilles (9) étant situé en aval dudit premier ensemble de lentilles (8) et étant adapté pour être déplacé par lesdits moyens de communication de mouvement (4) pour se rapprocher et s'éloigner dudit premier ensemble optique (8) et pour changer le zoom optique global ; un troisième ensemble de lentilles (10) situé en aval dudit deuxième ensemble de lentilles (9) et adapté pour être déplacé par lesdits éléments de communication de mouvement (4) pour se rapprocher et s'éloigner dudit deuxième ensemble optique (9) et pour régler la mise au point ; **caractérisé en ce que** le dispositif optique comprend en outre un quatrième ensemble de lentilles (11) pour focaliser le flux optique provenant du troisième ensemble de lentilles (10) sur lesdits éléments transducteurs (5) ; la distance entre lesdits premier (8) et quatrième (11) ensembles de lentilles étant invariable.

2. Endoscope selon la revendication 1, dans lequel ledit deuxième ensemble optique (9) comprend une lentille positive à ménisque (9a) et une lentille négative à doublet (9b) et a une distance focale globale négative.

3. Endoscope selon la revendication 1 ou 2, dans lequel ledit troisième ensemble de lentilles (10) comprend une lentille positive à ménisque (10a) et une lentille négative à doublet (10b) et a une distance focale globale positive.

4. Endoscope selon une quelconque revendication précédente, dans lequel ledit premier ensemble de lentilles (8) comprend une lentille négative à doublet (8a) et une lentille positive à ménisque (8b) et a une distance focale positive.

5. Endoscope selon une quelconque revendication précédente, dans lequel ledit quatrième ensemble de lentilles (11) comprend une lentille négative à doublet (11a), une lentille négative à ménisque (11b) et une lentille positive à doublet (11c) et a une distance focale positive.

6. Endoscope selon une quelconque revendication précédente dans lequel, dans chaque ensemble de lentilles (8, 9, 10, 11), les lentilles ont des diamètres égaux ou croissants de manière monotone, de la lentille la plus en amont à la lentille la plus en aval, par rapport au flux optique d'entrée, et inversement.

7. Endoscope selon une quelconque revendication précédente, comprenant un support (12, 13, 14, 15) pour chaque ensemble de lentilles (8, 9, 10, 11), ayant une pluralité de sièges (12a, 13a, 14a, 15a), chacun conçu pour recevoir et maintenir en position une lentille de l'ensemble de lentilles respectif.

8. Endoscope selon la revendication 7, dans lequel les sièges (12a, 13a, 14a, 15a) de chaque support (12, 13, 14, 15), qui sont conçus pour recevoir la lentille la plus en amont ou la plus en aval, comprennent un épaulement de retenu (12b, 13b, 14b, 15b) qui est conçu pour recevoir une portion de surface de la lentille en butée contre celui-ci.

9. Endoscope selon la revendication 7, comprenant des tiges de guidage (16) utilisables sur tous les supports (12, 13, 14, 15) ; lesdites tiges de guidage (16) retenant mécaniquement et de manière inamovible entre elles le support (12) du premier ensemble de lentilles (8) et le support (15) du quatrième ensemble de lentilles (11) et retenant de manière coulissante entre elles, et par rapport aux autres supports, le support (13) du deuxième ensemble de lentilles (9) et le support (14) du troisième ensemble de lentilles (10).

10. Endoscope selon une quelconque revendication précédente, dans lequel lesdits éléments de communication de mouvement (4) comprennent au moins un premier actionneur piézoélectrique linéaire (17) utilisable sur le deuxième ensemble de lentilles (9) et au moins un deuxième actionneur piézoélectrique linéaire (18) utilisable sur le troisième ensemble de lentilles (9) ; lesdits premier (17) et deuxième (18) actionneurs piézoélectriques étant agencés dans ledit boîtier (2) en aval du quatrième ensemble de lentilles (11).

11. Endoscope selon la revendication 10, dans lequel les premier (17) et deuxième (18) actionneurs piézoélectriques comprennent des pousseurs respectifs (17a, 18a), utilisables sur un support (13, 14) respectivement du deuxième ensemble de lentilles (9) et du troisième ensemble de lentilles (10) pour déplacer indépendamment le deuxième (9) et le troisième (10) ensemble de lentilles vers le premier ensemble de lentilles (8).

12. Endoscope selon la revendication 11, dans lequel lesdits pousseurs (17a, 18a) sont engagés mécaniquement à partir desdits supports (13, 14) ; lesdits pousseurs (17a, 18a) transférant des forces auxdits supports (13, 14) qui sont orientées seulement axialement, par rapport à l'axe suivant lequel les pousseurs (17a, 18a) s'étendent.

13. Endoscope selon la revendication 11 ou 12, dans lequel lesdits éléments de communication de mouvement (4) comprennent des éléments élastiques (19), utilisables sur un support (13, 14) du deuxième ensemble de lentilles (9) et du troisième ensemble de lentilles (10), pour pousser indépendamment le deuxième (9) et le troisième (10) ensemble de lentilles vers le quatrième ensemble de lentilles (11).

14. Endoscope selon la revendication 1, dans lequel l'unité de commande et de surveillance (8) est en communication de signal avec un système d'acquisition et de traitement (29) qui est conçu pour mettre en oeuvre un algorithme d'analyse de contraste d'image en temps réel, par le biais d'un processus de traitement d'image intermédiaire sur la base d'une maximisation de contraste.

15. Endoscope selon la revendication 14, dans lequel ledit processus intermédiaire comprend les étapes suivantes :
- la fourniture d'une k^{ième} image détectée par les éléments transducteurs,
- la sélection d'un ROI et de points distribués dans ladite image ;
- pour chaque point, le calcul de la différence de sa luminosité par rapport à celle des pixels voisins ;
- l'addition de ces différences pour générer un indice de contraste (Ck) de la k^{ième} image ;
- la comparaison de cet indice de contraste (Ck) avec une valeur de référence (Co),
- la maximisation du contraste de ladite image acquise en fonction de cette comparaison.
